(19)

Europäisches Patentamt

European Patent Office

Office européen des brevets

(11) **EP 3 321 273 B1**

(12) **EUROPEAN PATENT SPECIFICATION**

(45) Date of publication and mention
of the grant of the patent:
**23.02.2022 Bulletin 2022/08**

(21) Application number: **16871750.2**

(22) Date of filing: **30.08.2016**

(51) International Patent Classification (IPC):
**C07H 17/07** $^{(2006.01)}$   **C07H 1/00** $^{(2006.01)}$

(52) Cooperative Patent Classification (CPC):
**C07H 1/00; C07H 17/07**

(86) International application number:
**PCT/CN2016/097361**

(87) International publication number:
**WO 2018/039923 (08.03.2018 Gazette 2018/10)**

(54) **PREPARATION METHOD OF DIOSMIN**

HERSTELLUNGSVERFAHREN FÜR DIOSMIN

PROCÉDÉ DE PRÉPARATION DE DIOSMINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**16.05.2018 Bulletin 2018/20**

(73) Proprietor: **Chengdu Okay Pharmaceutical Co.,
Ltd
Qionglai City, Sichuan 611530 (CN)**

(72) Inventor: **ZHAO, Zhuojun
Qionglai
Sichuan 611530 (CN)**

(74) Representative: **Zwicker, Jörk
ZSP Patentanwälte PartG mbB
Hansastraße 32
80686 München (DE)**

(56) References cited:
**WO-A2-2010/092592      BE-A- 904 614
CN-A- 101 089 009      CN-A- 102 875 621
CN-A- 105 732 744      CN-A- 106 478 750**

Remarks:
The file contains technical information submitted after
the application was filed and not included in this
specification

## Description

### FIELD

The present invention relates to the field of drug synthesis technology, in particular to a method for preparing Diosmin.

### BACKGROUND

The chemical name of Diosmin is 3',5,7-trihydroxy-4'-methoxyflavone, i.e., (7-{[6-O-(6-deoxy-$\alpha$-L-mannose)-$\beta$-D-glucopyranose]oxy}-5-hydroxy-2-(3-hydroxy-4-metho xybenzene) -4H-L-benzopyran-4-one).

Diosmin has a comprehensive effect on the blood vessel reinfusion system, also strong effects on the venous system, the microcirculation system and the lymphatic system. Diosmin not only has the ability to significantly reduce the adhesion of leukocytes to vascular endothelial cells and the migration of leukocytes, inhibit the disintegration of leukocytes and the release of inflammatory mediators such as histamine, bradykinin, complement, leukotriene, prostaglandins, remove free radicals, but can also reduce blood viscosity and enhance flow rate of erythrocytes, in order to reduce the stasis of microcirculation. It is mainly used for the treatment of chronic venous insufficiency in clinic.

The content of Diosmin in natural plants is very low, and the cost of direct extraction is extremely high, so it is usually prepared by chemical synthesis of hesperidin oxidation.

In the methods for preparing Diosmin in the prior art, pyridine or morpholine is inevitably used as the reaction solvent, especially pyridine as the main. Since pyridine and morpholine as reaction solvents are not easy to be removed completely in the post-process procedure, the solvent residue caused in the product is high. Also, pyridine is a second-class organic solvent, with remarkably irritant odor and a relatively great toxic effect on human body, so the harm to the environment and personnel during the production process is more obvious.

Various processes for the preparation of Diosmin from Hesperidin are disclosed in BE904614 A, CN102875621 A, CN105732744 A, CN101089009 A and WO2010092592 A2.

### SUMMARY

In view of the above, the technical problem to be solved by the present invention is to provide a method for preparing Diosmin, which is environmentally friendly and by which a high purity of Diosmin can be prepared.

The present invention provides a method for preparing Diosmin, comprising:

A) mixing an amide solvent, hesperidin, an alkaline reagent and iodine, and performing a heating reaction to obtain Diosmin.

Preferably, the ratio of the amide solvent to hesperidin is (4~6)L: 1kg.

The mass ratio of iodine to hesperidin is (0.4~0.6):1.

The mass ratio of the alkaline reagent to hesperidin is (0.03~0.07):1.

Preferably, the step A) specifically consists of:

A1) mixing an amide solvent, hesperidin and an alkaline reagent to obtain a raw material solution;
A2) mixing the raw material solution and iodine, and performing a heating reaction to obtain Diosmin.

Preferably, the step A1) specifically consists of:
adjusting the pH of the amide solvents to 6-7 by an alkaline reagent, then adding hesperidin and mixing to obtain a raw material solution.

Preferably, the step A) further comprises:
recovering solvent under reduced pressure after heating reaction, adding water, filtering after crystallization, to obtain Diosmin.

Preferably, the temperature of recovering solvent under reduced pressure is 90°C~110°C.

The amide solvent is selected from methylacetamide, ethylacetamide, dimethyloxamide, dimethylacetamide or dimethylformamide.

The temperature of the heating reaction is 90 °C; the time of the heating reaction is 13~15h.

The present invention provides a method for preparing Diosmin, comprising: A) mixing an amide solvent, hesperidin, an alkaline reagent and iodine, and performing a heating reaction to obtain Diosmin. Compared with the prior art, an amide solvent is used as reaction solvent in the present invention, which are less toxic and soluble in water, and easy to be processed, so that the residual amount of solvent in the obtained product is reduced.

Experiments showed that the yield of Diosmin prepared by the present invention can be up to 85%~87% and HPLC purity is equal to or more than 92.5%.

## DETAILED DESCRIPTION OF THE INVENTION

The technical solutions in the embodiments of the present invention will be described clearly and completely in conjunction with the embodiments of the present invention.

The present invention provides a method for preparing Diosmin, comprising: A) mixing an amide solvent, hesperidin, an alkaline reagent and iodine, and performing a heating reaction to obtain Diosmin.

The source of all raw materials in the present invention is not particularly limited and it may be commercially available.

Wherein, the amide solvent can be one well-known to those skilled in the art and is not particularly limited. In the present invention, methylacetamide, ethylacetamide, dimethyloxamide, dimethylacetamide or dimethylformamide is preferred, and dimethylformamide is more preferred. The ratio of the amide solvent to hesperidin is preferably (4~6)L:1kg, more preferably (5~6)L:1kg. The alkaline reagent in the present invention are alkali metal hydroxides, and sodium hydroxide is preferred. The alkaline reagent is preferably a first-grade industrial product having a content of more than 99%. The mass ratio of the alkaline reagent to hesperidin is (0.03~0.07):1, preferably (0.04~0.06):1. The iodine is preferably the iodine having a content of more than 99.5%. The mass ratio of the iodine to hesperidin is (0.4~0.6):1, preferably 0.5:1.

Preferably, in the present invention, the step A) specifically consists of: A1) mixing an amide solvent, hesperidin and an alkaline reagent to obtain a raw material solution; A2) mixing the raw material solution and iodine, and performing a heating reaction to obtain Diosmin.

An amide solvent, hesperidin and an alkaline reagent are mixed. Preferably, hesperidin is added after the mixing of an amide solvent and an alkaline reagent. More preferably, the pH of the amide solvent is adjusted to 6-7 with an alkaline reagent and then hesperidin is added to obtain the raw material solution. In the present invention, it is preferable to stir the hesperidin while being added to make the materials sufficiently in contact with the solvent.

The raw material solution mentioned above is mixed with iodine and a heating reaction is carried out at 90 °C. the time of the heating reaction is preferably 13~15h, more preferably 14h.

After the completion of the heating reaction, solvents are recovered preferably under reduced pressure. The temperature of reaction solution is preferably controlled at 90°C~110°C, more preferably 90°C~100°C during reduced pressure recovery of solvent. The temperature and recovery situations are observed at any time during the recovery procedure. Preferably, the recovery of solvent is stopped when the reaction solution presents a thin-thick appearance. The recovery amount of the solvent is preferably 3.4~3.8 folds of the weight of hesperidin, more preferably 3.6~3.8 folds, even more preferably 3.7 folds.

In the present invention, an amide solvent is used as solvent for the oxidation reaction, which is easily to be recovered and reused, and has a relatively small pollution to the environment. Compared with the traditional pyridine or morpholine solvent, the toxicity of an amide solvent is lower, and the solvent residue in the resultant product is low, soluble in water, easy to handle, and solvent residue in the product can be controlled within 10ppm in the product. Also, during the reaction process from hesperidin to Diosmin, there is less degradation products, making the impurity content in the product low. The amide solvents have a higher boiling point than that of pyridine or morpholine, so less volatile under the same reaction conditions.

After the completion of the solvent recovery, water is added for crystallization. This process is preferably carried out by adding water to the thin-thick reaction solution and stirring; more preferably, more preferably carried out by adding water to the thin-thick reaction solution and stirring for 10~20min and adding water again and stirring for 30~90min .

Water is added for crystallization. Preferably, the temperature of crystalline solution is reduced to 30°C~40°C, more preferably to 33°C~37°C, even more preferably to 35°C; then filtration is performed.

After filtration, preferably, filter cake is washed by water; more preferably, wash is carried out until the pH of liquid after washing the filter cake is 6-7, and wash is then completed.

After the completion of wash, preferably, the filter cake is subjected to air compression; the pressure of the air compression is preferably 0.07~0.09MPa; and the time of the air compression is preferably 2~4h, more preferably 2.5~3.5h, and even more preferably 3h.

In order to obtain Diosmin containing less impurities, it is preferable to perform a secondary dissolution filtration. Preferably, the filter cake after air compression is mixed with an alkaline solution. The alkaline solution is an aqueous solution of the alkaline reagent. The alkaline reagent in the alkaline solution is an alkaline reagent well-known to those skilled in the art and not particularly limited. In the present invention, it is preferably an alkali metal hydroxide, more preferably sodium hydroxide. The mass ratio of the alkaline reagent in the alkaline solution to hesperidin is preferably (0.1~0.5):1, more preferably (0.2~0.4):1, even more preferably 0.3:1. The time of the mixing is preferably 0.5~2h, more preferably 1~1.5h, and even more preferably 1h.

After mixing, water is preferably added again and stirred. Time of stirring is preferably 10~60min, more preferably 20~50min, even more preferably 30~40min, and most preferably 30min.

After stirring, the mixture is allowed to stand for 2~6h preferably, more preferably 3~5h, even more preferably 3~4h, and most preferably 3h. Filtration is performed after standing, so as to obtain filtrate.

In the present invention, in order to reduce the impurities, it is preferable to filter the filtrate with a fine filter to obtain a fine filtrate.

After the fine filtration, a secondary crystallization is performed. Preferably, in the present invention, the fine filtrate is mixed with hydrochloric acid. The mass ratio of the hydrochloric acid to hesperidin is preferably (0.64~0.92):1. The concentration of the hydrochloric acid is preferably 30%~40%, more preferably 35%~40%, even more preferably 36%. Preferably, in the present invention, hydrochloric acid is added to adjust the pH of the filtrate to 5-6. The time of the mixing is preferably 1~2h, more preferably 1~1.5h.

After mixing, the mixture is filtered to obtain a filter cake. Preferably, the filter cake is washed with water and dried to obtain Diosmin. The temperature of the drying is preferably 100°C~130°C and the time of the drying is preferably 10~16h. After drying for 10h, samples are taken at appropriate times and water content is measured by a rapid moisture meter. When water content in the sample is less than 5%, drying is stopped.

In the present invention, an amide solvent is used as reaction solvent, which is less toxic and soluble in water, and easy to be processed, so that the residual amount of solvent in the obtained product is reduced.

In order to further illustrate the present invention, the method for preparing Diosmin provided in the present invention will be described in detail below with reference to the following examples.

The reagents used in the following examples are all commercially available.

**Example 1**

1.1 Oxidation reaction

A vacuum pump was turned on and 1500L dimethylformamide was sucked into a reaction tank in vacuum, and sodium hydroxide was added to adjust the pH of the solvent to 6-7. 250.00kg of hesperidin was added and stirred while feeding to ensure the fully contact of the material and solvent. 125kg of iodine was added to the reaction tank at a constant speed and reacted. The temperature of reaction tank was controlled at 70°C~100°C and the reaction was carried out for 14h.

1.2 Recovery of solvent

After the reaction was completed, the valves of a transfer tank and a dewatering tank were opened and the return valve was closed. The material temperature was controlled between 90°C and 110°C, and the solvent was recovered under reduced pressure. The temperature and recovery situation were observed at any time during the recovery procedure. When the materials presented a thin-thick appearance, recovery of solvent was stopped to enter the next step.

1.3 Crystallization, filtration and washing of the crude product

The vacuum valve of the reaction tank was opened and 1500L of purified water was drawn from the upper part of the reaction tank. The stirrer was operated, stirred for 10-20 min, then 1500L of purified water was added and stirred for 1h. After the stirring, when the temperature of crystalline solution dropped to 35°C, the crystalline solution was pumped into a frame filter for filtration and the filtrate was temporarily stored in the storage tank. When there was no liquid outflow from the nozzle of the frame filter after filtration, 18,000L purified water was pumped into the frame filter for washing. The washing solution was discharged into the anhydrous collection pool for sewage treatment. Washing was carried out until the pH of the effluent water was 6-7 by using pH test paper for detection and the washing solution should be colorless, clear and transparent by beaker sampling observation. After the completion of washing, the inlet valve was closed and the air pressure valve was opened for air compression; the pressure of air compression was controlled at 0.07~0.09MPa, and the time was maintained for 3h. Filter cake was collected, put into the transfer tank and labeled correctly.

1.4 Secondary dissolution and filtration

The dissolving tank was opened and stirred. In the dissolving tank, 75kg of base A was added to 1300L of purified water; after being completely dissolved, the filter cake in the transfer tank was added to the dissolving tank. After adding the filter cake and continuing stirring for 1h, 1300L of purified water was added to the dissolving tank and stirred for 30min, and stood for 3h. The filtrate was filtered once by the frame filter, and the filtered solution was filtered by the fine filter and pumped into a crystallization tank in the clean area.

1.5 Secondary crystallization

The stirrer was started, and 160~230kg of 36% hydrochloric acid was slowly placed into the crystallization tank in the clean area and the pH of solution was measured to be between 5.0 and 6.0. After stirring for 20min, the pH of the solution should be stable and qualified. The solution was stirred for 1h. The original record of the process was done.

1.6 Filtration and washing of the fine product

The crystalline solution was sucked into a chamber filter press through vacuum inhalation for filtration. After filtration, the product was washed with 18,000~20,000L of purified water. After washing, the washing solution was checked to ensure that it was colorless, clear and transparent. The filtrate was discharged into the sewage treatment system.

1.7 Drying of the fine product

The hot air circulation oven was turned on, the temperature was controlled at 100°C~130°C, and the drying time was maintained at 10~16h. After drying for 10h, samples were taken at appropriate times and water content was measured by a rapid moisture meter, and when the water content was less than 5%, drying was stopped. After drying was completed, the oven was closed and the material was bagged and transferred to the next step.

1.8 Pulverization of fine product

The dried product was pulverized with a pulverizer, and passed an 80-mesh sieve to obtain Diosmin.
The quality standards of all the materials in Example 1 were shown in Table 1.

Table 1. Quality standards of raw materials and adjuvants.

| Name of raw materials and adjuvants | Grade | Quality standard |
| --- | --- | --- |
| Hesperidin | unified goods | meet "Hesperidin quality standard" |
| new solvent | industrial grade | meet "new solvent quality standard" |
| base A | industrial grade | meet "base A quality standard" |
| Iodine | medicinal grade | meet "Iodine quality standard" |
| hydrochloric acid | analytically pure | meet "hydrochloric acid quality standard" |

Method of calculating the recovery rate of Diosmin:

[0057]     Diosmin recovery rate $=$(weight of fine Diosmin)/(weight of hesperidin) $\times 100\%$

**Example 2**

According to the steps in Example 1, orthogonal experiments were set up, and the reaction temperature (A) and the reaction time (B) were selected as two factors. Factor A was set at four levels and factor B was set at three levels. Factor levels were shown in Table 2. Orthogonal experiment design was used to screen the oxidative reaction conditions of Diosmin using Diosmin content, total impurity and recovery rate as three investigation items. The results of orthogonal experiments were shown in Table 3 and the analysis of results of orthogonal experiments was shown in Table 4.

Table 2. Factor levels of orthogonal experiments.

| level | A/°C | B/h |
|-------|------|-----|
| 1 | 70 | 13 |
| 2 | 80 | 14 |
| 3 | 90 | 15 |
| 4 | 100 | -- |

Table 3. The results of orthogonal experiments

| number | A | B | content (%) | total impurity (%) | recovery rate (%) |
|--------|-----|-----|-------------|--------------------|--------------------|
| 1 | 70 | 13 | 92.3 | 6.32 | 84.6 |
| 2 | 70 | 14 | 92.7 | 6.45 | 85.1 |
| 3 | 70 | 15 | 92.4 | 5.24 | 84.3 |
| 4 | 80 | 13 | 93.6 | 5.31 | 85.3 |
| 5 | 80 | 14 | 93.2 | 4.82 | 85.4 |
| 6 | 80 | 15 | 93.1 | 5.11 | 85.6 |
| 7 | 90 | 13 | 94.1 | 4.41 | 84.4 |
| 8 | 90 | 14 | 94.8 | 4.32 | 85.2 |
| 9 | 90 | 15 | 94.3 | 4.12 | 84.7 |
| 10 | 100 | 13 | 92.2 | 5.29 | 86.2 |
| 11 | 100 | 14 | 92.6 | 5.66 | 86.0 |
| 12 | 100 | 15 | 92.5 | 6.05 | 86.3 |

Table 4. Analysis of the results of orthogonal experiments

| T value | content (%) | | total impurity (%) | | recovery rate (%) | |
|---------|------|------|------|------|------|------|
| | A | B | A | B | A | B |
| $T_1$ | 277.4 | 372.2 | 18.01 | 21.33 | 254 | 340.5 |
| $T_2$ | 279.9 | 373.3 | 15.24 | 21.25 | 256.3 | 341.7 |
| $T_3$ | 283.2 | 372.3 | 12.85 | 20.52 | 254.3 | 340.9 |
| $T_4$ | 277.3 | | 17.0 | | 258.5 | |
| $t_1$ | 92.47 | 93.05 | 6.00 | 5.33 | 84.67 | 85.13 |
| $t_2$ | 93.30 | 93.32 | 5.08 | 5.31 | 85.43 | 85.43 |
| $t_3$ | 94.40 | 93.08 | 4.28 | 5.13 | 84.77 | 8 5.2 3 |
| $t_4$ | 92.43 | | 5.67 | | 86.17 | |
| range | 1.97 | 0.27 | 1.72 | 0.20 | 1.50 | 0.20 |
| priority level | $A_3$ | $B_2$ | $A_3$ | $B_3$ | $A_4$ | $B_2$ |
| primary and secondary factors | AB | | AB | | AB | |

**EP 3 321 273 B1**

Based on comprehensive analysis, the best combinations were $A_3B_2$, $A_3B_3$, and $A_4B_2$. Among these three best combinations, based on content as the main consideration, $A_3B_2$ combination was selected. At the condition of $A_3B_2$, the content of Diosmin reached the highest value and the total impurity reached the lowest value, that is, the optimum conditions for Diosmin oxidation reaction were as follows: reaction temperature 90°C, reaction time 14 hours.

**Example 3**

According to the steps in Example 1, orthogonal experiments were set up. Recovery temperature of solvent and the amount ratio of the recovered solvent were selected as single factor, respectively. Each factor was set at five levels and each level was test three times, with Diosmin content, total impurity and recovery rate as three investigation items. The results of experiments were shown in Table 5 and Table 6. The conditions for solvent recovery of Diosmin synthesis were screened according to the results of statistical test of the experiments.

Table 5. The results of three trials of single factor (recovery temperature) with multiple levels.

| recovery temperature (°C) | content (%) (three trials) | | | | total impurity (%) (three trials) | | | | recovery rate (%) (three trials) | | | |
|---|---|---|---|---|---|---|---|---|---|---|---|---|
| | ① | ② | ③ | average | ① | ② | ③ | average | ① | ② | ③ | average |
| 90 | 92.3 | 92.5 | 92.6 | 92.57 | 4.68 | 4.75 | 4.59 | 4.67 | 85.6 | 85.2 | 85.4 | 85.40 |
| 95 | 93.7 | 93.4 | 93.3 | 93.47 | 4.11 | 4.26 | 4.32 | 4.23 | 86.1 | 86.3 | 85.4 | 85.93 |
| 100 | 94.0 | 94.2 | 94.5 | 94.23 | 5.02 | 5.12 | 5.08 | 5.07 | 86.3 | 86.1 | 86.5 | 86.30 |
| 105 | 93.6 | 93.2 | 93.5 | 93.43 | 5.42 | 5.26 | 5.37 | 5.35 | 85.3 | 85.2 | 85.5 | 85.33 |
| 110 | 92.2 | 92.4 | 92.3 | 92.30 | 5.76 | 5.88 | 6.03 | 5.89 | 85.4 | 85.3 | 85.5 | 85.40 |

Table 6. The results of three trials of single factor (the amount ratio of recovered solvent) with multiple levels.

| the amount ratio of recovered solvent (fold) | content (%) (three trials) | | | | total impurity (%) (three trials) | | | | recovery rate (%) (three trials) | | | |
|---|---|---|---|---|---|---|---|---|---|---|---|---|
| | ① | ② | ③ | average | ① | ② | ③ | average | ① | ② | ③ | average |
| 3.4 | 92.4 | 92.7 | 92.5 | 92.53 | 4.06 | 4.17 | 4.21 | 4.15 | 85.1 | 85.2 | 85.4 | 85.23 |
| 3.5 | 92.6 | 92.8 | 92.5 | 92.63 | 4.66 | 4.78 | 4.59 | 4.68 | 85.7 | 85.3 | 85.5 | 85.50 |
| 3.6 | 93.7 | 93.2 | 93.6 | 93.50 | 5.13 | 5.31 | 5.22 | 5.22 | 86.1 | 86.4 | 86.1 | 86.20 |
| 3.7 | 94.2 | 94.2 | 94.4 | 94.27 | 5.49 | 5.67 | 5.58 | 5.58 | 85.3 | 85.2 | 85.6 | 85.37 |
| 3.8 | 93.1 | 92.9 | 92.8 | 92.93 | 5.92 | 5.98 | 6.05 | 5.98 | 85.4 | 85.8 | 85.7 | 85.63 |

Based on the comprehensive analysis of the above table, when the Diosmin content and recovery rate reached the highest value, and the total impurity reached the lowest value, the solvent recovery temperatures were 100°C, 100°C, 95°C, respectively, and the amount ratios of recovered solvent were 3.7-fold, 3.6-fold, 3.4-fold, respectively. All the results met the quality standards. When content of Diosmin was used as the main investigation item, the optimum conditions for the recovery of Diosmin solvent were as follows: recovery temperature 100°C, and the amount ratio of recovered solvent 3.7-fold.

**Example 4**

According to the steps in Example 1, orthogonal experiments were set up. The ratios by weight of new solvent to hesperidin, base A to hesperidin, iodine to hesperidin were selected as three factors A, B, C, respectively. Each factor was set at three levels and the factor levels were shown in Table 7. The ratios of raw materials and adjuvants used were screened by $L_9(3^3)$ orthogonal experiments using Diosmin content, total impurity and recovery rate as three investigation items. Data statistical analysis was shown in Table 8 and Table 9.

Table 7. Factor levels of orthogonal experiment in Example 4.

| Level | A (fold) | B (fold) | C (fold) |
|---|---|---|---|
| 1 | 4 | 0.03 | 0.4 |
| 2 | 5 | 0.05 | 0.5 |
| 3 | 6 | 0.07 | 0.6 |

Table 8. The results of orthogonal experiments in Example 4.

| number | A | B | c | content (%) | total impurity (%) | recovery rate (%) |
|---|---|---|---|---|---|---|
| 1 | 4 | 0.03 | 0.4 | 92.5 | 5.42 | 85.1 |
| 2 | 4 | 0.05 | 0.5 | 93.1 | 5.23 | 85.4 |
| 3 | 4 | 0.07 | 0.6 | 92.7 | 5.56 | 85.2 |
| 4 | 5 | 0.03 | 0.6 | 93.2 | 4.68 | 85.1 |
| 5 | 5 | 0.05 | 0.4 | 93.4 | 4.43 | 85.5 |
| 6 | 5 | 0.07 | 0.5 | 93.5 | 4.35 | 85.7 |
| 7 | 6 | 0.03 | 0.5 | 94.4 | 4.98 | 86.6 |
| 8 | 6 | 0.05 | 0.6 | 93.8 | 5.12 | 86.2 |
| 9 | 6 | 0.07 | 0.4 | 94.2 | 5.06 | 86.1 |

Table 9. Analysis of the results of orthogonal experiments in Example 4.

| T value | content (%) | | | total impurity (%) | | | recovery rate (%) | | |
|---|---|---|---|---|---|---|---|---|---|
| | A | B | C | A | B | C | A | B | C |
| $T_1$ | 278.3 | 280.1 | 280.1 | 16.21 | 15.08 | 14.91 | 255.7 | 256.8 | 256.7 |
| $T_2$ | 280.1 | 280.3 | 281.0 | 13.46 | 14.78 | 14.56 | 256.3 | 257.1 | 257.7 |
| $T_3$ | 282.4 | 280.4 | 279.7 | 15.16 | 14.97 | 15.36 | 258.9 | 257.0 | 256.5 |
| $t_1$ | 92.77 | 93.37 | 93.37 | 5.40 | 5.03 | 4.97 | 85.23 | 85.60 | 85.57 |
| $t_2$ | 93.37 | 93.43 | 93.67 | 4.49 | 4.93 | 4.85 | 85.43 | 85.70 | 85.90 |
| $t_3$ | 94.13 | 93.47 | 93.23 | 5.05 | 4.99 | 5.12 | 86.30 | 85.67 | 85.50 |
| range | 1.36 | 0.10 | 0.47 | 0.91 | 0.10 | 0.27 | 1.07 | 0.10 | 0.40 |
| priority level | $A_3$ | $B_3$ | $C_2$ | $A_2$ | $B_2$ | $C_2$ | $A_3$ | $B_2$ | $C_2$ |

(continued)

| T value | content (%) | | | total impurity (%) | | | recovery rate (%) | | |
|---|---|---|---|---|---|---|---|---|---|
| | A | B | C | A | B | C | A | B | C |
| primary and secondary factors | ACB | | | ACB | | | ACB | | |

Based on comprehensive analysis, the best combinations were $A_3B_3C_2$, $A_2B_2C_2$ and $A_3B_2C_2$. Among these three best combinations, based on the Diosmin content as the main consideration, $A_3B_2C_2$ combination was selected. At the condition of $A_3B_2C_2$, the Diosmin content and recovery rate reached the highest value, and the total impurity reached the lowest value, that is, the optimum ratios of raw materials and adjuvants used were as follows: dimethylformamide:hesperidin=6:1; for oxidation reaction, sodium hydroxide:hesperidin = 0.05:1, and iodine:hesperidin = 0.5:1.

## Claims

1. A method for preparing Diosmin, comprising:

    A) mixing an amide solvent, hesperidin, an alkaline reagent and iodine, and performing a heating reaction at 90°C to obtain Diosmin,

       wherein the amide solvent is selected from methylacetamide, ethylacetamide, dimethyloxamide, dimethylacetamide or dimethylformamide,
       wherein the alkaline reagent is alkali metal hydroxide,
       wherein the mass ratio of the alkaline reagent to hesperidin is (0.03-0.07):1,
       wherein the mass ratio of the iodine to hesperidin is (0.4-0.6):1.

2. The method according to claim 1, **characterized in that**, the ratio of the amide solvent to hesperidin is (4-6)L:1kg.

3. The method according to claim 1, **characterized in that**, the step A) specifically consists of:

    A1) mixing an amide solvent, hesperidin and an alkaline reagent to obtain a raw material solution; and
    A2) mixing the raw material solution and iodine, and performing a heating reaction to obtain Diosmin.

4. The method according to claim 3, **characterized in that**, the step A1) specifically consists of:
   adjusting the pH of the amide solvent to 6-7 with an alkaline reagent, then adding hesperidin and mixing to obtain a raw material solution.

5. The method according to claim 1, **characterized in that**, the step A) further comprises:
   recovering solvent under reduced pressure after heating reaction, adding water, and filtering after crystallization, to obtain Diosmin.

6. The method according to claim 5, **characterized in that**, the temperature of recovering solvent under reduced pressure is 90°C-110°C.

7. The method according to claim 1, **characterized in that**, the alkali metal hydroxide is sodium hydroxide.

8. The method according to claim 1, **characterized in that** the time of the heating reaction is 13-15h.

## Patentansprüche

1. Verfahren zum Herstellen von Diosmin, umfassend:

    A) Vermengen eines Amidlösungsmittels, Hesperidin, einem alkalinen Reagenz und Iod, und Durchführen einer Hitzereaktion bei 90°C, um Diosmin zu erhalten,

wobei das Amidlösungsmittel ausgewählt ist unter Methylacetamid, Ethylacetamid, Dimethyloxamid, Dimethylacetamid oder Dimethylformamid,
wobei das alkaline Reagenz Alkalimetallhydroxid ist,
wobei das Masseverhältnis des alkalinen Reagenz zu Hesperidin (0.03-0.07):1 beträgt,
wobei das Masseverhältnis des Iods zu Hesperidin (0.4-0.6):1 beträgt.

2. Verfahren nach Anspruch 1, **dadurch gekennzeichnet, dass** das Verhältnis des Amidlösungsmittels zu Hesperidin (4-6)L:1kg beträgt.

3. Verfahren nach Anspruch 1, **dadurch gekennzeichnet, dass** der Schritt A) insbesondere besteht aus:

A1) Vermengen eines Amidlösungsmittels, Hesperidin und einem alkalinen Reagenz, um eine Lösung aus Rohmaterial zu erhalten; und
A2) Vermengen der Lösung aus Rohmaterial und Iod, und Durchführen einer Hitzereaktion bei 90°C, um Diosmin zu erhalten.

4. Verfahren nach Anspruch 3, **dadurch gekennzeichnet, dass** der Schritt A1) insbesondere besteht aus:
Einstellen des pH-Werts des Amidlösungsmittels auf 6-7 mit einem alkalinen Reagenz, dann Hinzufügen von Hesperidin und Vermengen, um eine Lösung aus Rohmaterial zu erhalten.

5. Verfahren nach Anspruch 1, **dadurch gekennzeichnet, dass** der Schritt A) ferner umfasst:

Rückgewinnen von Lösungsmittel unter verringertem Druck nach der Hitzereaktion,
Hinzufügen von Wasser und Filtrieren nach einer Kristallisation, um Diosmin zu erhalten.

6. Verfahren nach Anspruch 5, **dadurch gekennzeichnet, dass** die Temperatur bei der Rückgewinnung von Lösungsmittel unter verringertem Druck 90°C-110°C beträgt.

7. Verfahren nach Anspruch 1, **dadurch gekennzeichnet, dass** das Alkalimetallhydroxid Natriumhydroxid ist.

8. Verfahren nach Anspruch 1, **dadurch gekennzeichnet, dass** die Dauer der Hitzereaktion 13-15h beträgt.


**Revendications**

1. Procédé pour préparer de la diosmine, comprenant l'étape :

A) consistant à mélanger un solvant de type amide, de l'hespéridine, un réactif alcalin et de l'iode, et à effectuer une réaction de chauffage à 90 °C pour obtenir de la diosmine,

dans lequel le solvant de type amide est choisi parmi le méthylacétamide, l'éthylacétamide, le diméthyloxamide, le diméthylacétamide et le diméthylformamide,
dans lequel le réactif alcalin est un hydroxyde de métal alcalin,
dans lequel le rapport en masse du réactif alcalin à l'hespéridine est (0,03-0,07) :1,
dans lequel le rapport en masse de l'iode à l'hespéridine est (0,4-0,6):1.

2. Procédé selon la revendication 1, **caractérisé en ce que** le rapport du solvant de type amide à l'hespéridine est (4-6) L:1 kg

3. Procédé selon la revendication 1, **caractérisé en ce que** l'étape A) consiste précisément à :

A1) mélanger un solvant de type amide, de l'hespéridine et un réactif alcalin pour obtenir une solution de substance brute ; et
A2) mélanger la solution de substance brute et de l'iode, et effectuer une réaction de chauffage pour obtenir de la diosmine.

4. Procédé selon la revendication 3, **caractérisé en ce que** l'étape A) consiste précisément à :
ajuster le pH du solvant de type amide à 6-7 à l'aide d'un réactif alcalin, puis ajouter de l'hespéridine et mélanger pour obtenir une solution de substance brute.

5. Procédé selon la revendication 1, **caractérisé en ce que** l'étape A) comprend en outre :
récupérer le solvant sous pression réduite après la réaction de chauffage, ajouter de l'eau, et filtrer après cristallisation, pour obtenir de la diosmine.

6. Procédé selon la revendication 5, **caractérisé en ce que** la température de récupération du solvant sous pression réduite est de 90 °C - 110 °C.

7. Procédé selon la revendication 1, **caractérisé en ce que** l'hydroxyde de métal alcalin est l'hydroxyde de sodium.

8. Procédé selon la revendication 1, **caractérisé en ce que** la durée de la réaction de chauffage est de 13-15 h.

**EP 3 321 273 B1**

## REFERENCES CITED IN THE DESCRIPTION

### Patent documents cited in the description

- BE 904614 A
- CN 102875621 A
- CN 105732744 A
- CN 101089009 A
- WO 2010092592 A2